(19) Europäisches Patentamt
European Patent Office
Office européen des brevets

(11) Publication number: **0 201 198**
**B1**

# (12) EUROPEAN PATENT SPECIFICATION

(45) Date of publication of patent specification: **09.08.89**

(51) Int. Cl.$^4$: **C 07 D417/12**, A 61 K 31/44

(21) Application number: **86302496.4**

(22) Date of filing: **04.04.86**

## (54) Pyridine derivatives.

(30) Priority: **11.04.85 GB 8509277**

(43) Date of publication of application:
**12.11.86 Bulletin 86/46**

(45) Publication of the grant of the patent:
**09.08.89 Bulletin 89/32**

(84) Designated contracting states:
**AT BE CH DE FR GB IT LI LU NL SE**

(56) References cited:
**EP–A– 0 112 704**
**EP–A– 0 194 746**
**W. KERN: "Hagers Handbuch der Pharmazeutischen Praxis", vol. 1, 4th edition, 1967, pages 1177-1183, Springer-Verlag, Berlin, DE**
**The file contains technical information submitted after the application was filed and not included in this specification**

(73) Proprietor: **SMITH KLINE & FRENCH LABORATORIES LIMITED**
**Mundells**
**Welwyn Garden City Hertfordshire, AL7 1EY (GB)**

(72) Inventor: **Ife, Robert John**
**19 Mobbsbury Way**
**Stevenage Hertfordshire (GB)**

(74) Representative: **Waters, David Martin, Dr. et al**
**Smith Kline & French Laboratories Ltd. Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 201 198 B1

Jouve, 18, rue St-Denis, 75001 Paris, France

## Description

This invention relates to certain pyridine derivatives, a process for their preparation, compositions containing them and their use as histamine $H_1$-antagonists.

Histamine, a physiologically active compound endogenous in mammals, exerts its action by interacting with certain sites called receptors. On type of receptor is known as a histamine $H_1$-receptor (Ash and Schild, Brit. J. Pharmac. 1966, 27, 427) and the actions of histamine at these receptors are inhibited by drugs commonly called « antihistamines » (histamine $H_1$-antagnists) a common example of which is mepyramine.

European Patent Application EP 0 112 704 describes a class of histamine $H_1$-receptor antagonists having the general formula (A)

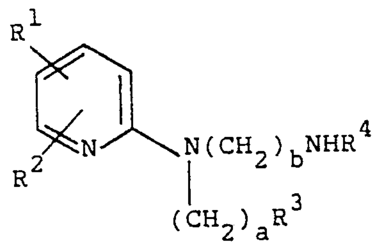

(A)

wherein $R^1$ and $R^2$ are the same or different and are hydrogen, $C_{1-6}$-alkyl, $C_{1-6}$-alkoxy or halogen ;

$R^3$ is optionally substituted phenyl or optionally substituted pyridyl, where the optional substituents are one or more $C_{1-6}$-alkyl, $C_{1-6}$-alkoxy, or hydroxy groups or halogen atoms ; or optionally substituted thiazolyl, optionally substituted furanyl or optionally substituted thienyl where the optional substituents are one or more $C_{1-6}$-alkyl or $C_{1-6}$-alkoxy groups or halogen atoms ;

a is from 1 to 6

b is from 2 to 4

and $R_4$ is one of several cyclic groups including the group (B)

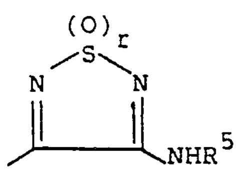

(B)

where $R^5$ is hydrogen, $C_{1-6}$-alkyl, or $(CH_2)_dR^6$ where d is 1-6 and $R^6$ is optionally substituted phenyl, optionally substituted pyridyl, where the optional substituent is one or more $C_{1-6}$-alkyl, $C_{1-6}$-alkoxy or hydroxy groups or halogen atoms ; optionally substituted thiazolyl, optionally substituted furanyl or optionally substituted thienyl, where the optional substituents are one or more $C_{1-6}$-alkyl or $C_{1-6}$-alkoxy groups or halogen atoms ; and r is 0 to 2.

European Patent Application No. 86300795.1 (Publication No. 0 194 746), which was published after the filing date of the present application, discloses as histamine $H_1$-receptor antagonists compounds of the formula (C)

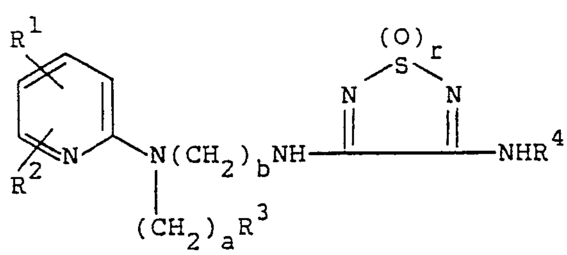

(C)

wherein $R^1$ and $R^2$ are the same or different and are hydrogen, $C_{1-6}$-alkyl, $C_{1-6}$-alkoxy or halogen ;

$R^3$ is optionally substituted phenyl or optionally substituted pyridyl, where the optional substituents are one or more $C_{1-6}$-alkyl, $C_{1-6}$-alkoxy, or hydroxy groups or halogen atoms ; or furanyl or thienyl optionally substituted by a $C_{1-6}$-alkyl group ;

a is from 1 to 6 ;

b is from 2 to 4 ;

$R^4$ is $CH_2R^5$ where $R^5$ is phenyl substituted by sulphonamido or a carboxyl group or an ester thereof,

pyridyl substituted with a carboxyl group or an ester thereof and optionally further substituted with a $C_{1-6}$-alkyl group, N-oxo-pyridyl optionally substituted with a $C_{1-6}$-alkyl group, or an imidazolyl, pyridazinyl, pyrimidinyl, pyrazinyl or benzimidazolyl group ; and

r is 1 or 2.

The present invention provides compounds of the formula (1)

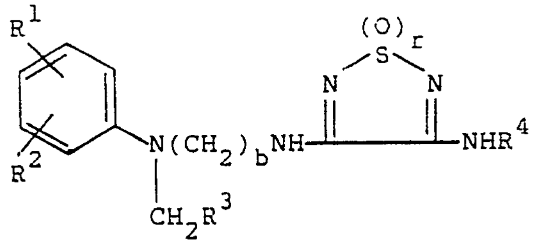

(1)

and pharmaceutically acceptable salts thereof, where

$R^1$ and $R^2$ are the same or different and are hydrogen, $C_{1-6}$-alkyl, $C_{1-6}$-alkoxy or halogen ;

$R^3$ is optionally substituted pyridyl, where the optional substituents are one or more $C_{1-6}$-alkyl, $C_{1-6}$-alkoxy, or hydroxy groups or halogen atoms ;

b is from 2 to 4

$R^4$ is hydrogen or $C_{1-6}$-alkyl, and

r is 1 or 2.

The compounds of this invention are histamine $H_1$-antagonists and are useful for the treatment of diseases for example bronchial asthma, rhinitis, hayfever and allergic eczema whose symptoms are mediated through the action of histamine at $H_1$-receptors.

Examples of halogens for $R^1$, $R^2$ and the halogen substituent in $R^3$ are fluorine, chlorine, bromine and iodine.

Examples of $C_{1-6}$-alkyl groups for $R^1$ and $R^2$ and the $C_{1-6}$-alkyl substituent in $R^3$ are methyl, ethyl, n-propyl, iso-propyl, n-butyl and t-butyl.

Examples of $C_{1-6}$-alkoxy groups for $R^1$ and $R^2$ and the $C_{1-6}$-alkoxy substituents in $R^3$ are methoxy, ethoxy, n-propoxy and n-butoxy.

Preferably $R^1$ and $R^2$ are both hydrogen.

By way of example, b can be 2, 3 or 4. Preferably it is 3.

Examples of optionally substituted pyridyl groups for $R^3$ are optionally substituted 2-pyridyl, 3-pyridyl or 4-pyridyl groups.

In particular $R^3$ is 2-pyridyl, 3-pyridyl or 4-pyridyl. Preferably $R^3$ is 2-pyridyl or 4-pyridyl.

$R^4$ is in particular, hydrogen or methyl. Preferably $R^4$ is hydrogen.

Preferably r is 1.

Compounds of formula (1) also form pharmaceutically acceptable salts with pharmaceutically acceptpable acid addition salt-forming acids. Accordingly, in this specification the term « pharmaceutically acceptable salts » means salts with bases or acid addition salts as the context requires.

Examples of pharmaceutically acceptable acid addition salt-forming acids are hydrochloric, sulphuric, hydrobromic, phosphoric, tartaric, citric, maleic, lactic, 2-hydroxyethanesulphonic, methanesulphonic, toluene-4-sulphonic, ethanedisulphonic, ethanesulphonic and camphorsulphonic acids.

Compounds of formula (1) can be prepared by reacting an amine of formula (2)

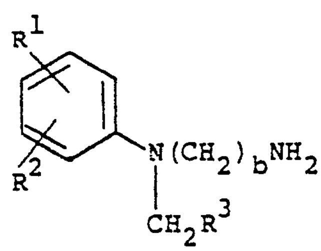

(2)

where $R^1$, $R^2$, $R^3$, and b are as defined with reference to formula (1) with a compound of formula (3)

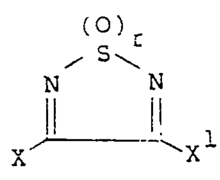

(3)

where X is a group displaceable with an amine and $X^1$ is a group of formula $NHR^4$ or a group displaceable with an amine, where $R^4$ is as previously defined ; and thereafter, where $X^1$ is a group displaceable with an amine, reacting with an amine of formula (4)

$$R^4NH_2 \qquad (4)$$

where $R^4$ is as defined with reference to formula (1).

Examples of leaving groups displaceable by amines are where X and $X^1$ are QS—, QSO—, $QSO_2$—, or QO— (Q being $C_{1-6}$-alkyl, aryl or aralkyl), halogen, particularly chlorine and bromine, and nitroamino. Preferably the groups X and $X^1$ are QO— where Q is methyl.

The conditions under which the reaction is carried out depends upon the nature of the reagents. The reaction is carried out at moderate to low temperature e. g. from 0 °C to room temperature. The choice of solvent is affected by the solubility characteristics of the reagents. Preferably the solvent is pyridine, a picoline or mixture of picolines, a $C_{1-6}$-alkanol, preferably ethanol or 1-propanol, 1,2-ethanediol, a high boiling alkoxyaryl ether for example anisole, or a polar aprotic solvent, for example dimethylformamide, dimethylacetamide, dimethylsulphoxide, hexamethylphosphoramide or sulpholane.

Pharmaceutically acceptable acid-addition salts of compounds of formula (1) can be prepared by standard methods, for example by reacting a solution of the compound of formula (1) with a solution of the acid.

Compounds of formula (2) can be prepared by reacting a compound of formula (5)

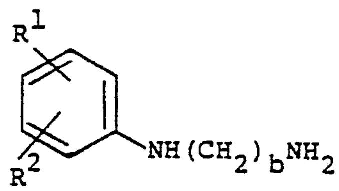

(5)

where $R^1$ and $R^2$ and b are as defined with reference to formula (1) or a derivative thereof where the primary amino group is protected, with a compound of formula (6)

$$R^3CH_2X^2 \qquad (6)$$

where $R^3$ is as defined with reference to formula (1) (provided that any hydroxy groups in $R^3$ are protected) and $X^2$ is halogen, in the presence of a strong base and thereafter removing any protecting groups.

Compounds of formula (5) can be prepared in turn by reducing a compound of formula (7)

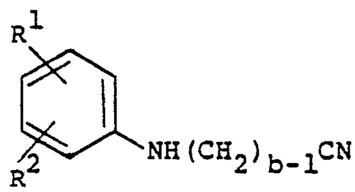

(7)

where $R^1$, $R^2$ and b are as defined with reference to formula (1).

Preferably the reducing agent is lithim aluminium hydride.

Compounds of formula (5) can also be prepared by reacting a compound of formula (8)

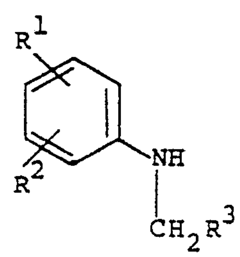

(8)

where $R^1$, $R^2$ and $R^3$ are as defined with reference to formula (1) (provided that any hydroxy groups in $R^3$ are protected) with a compound of formula (9)

$$X^2(CH_2)_bR^6 \quad (9)$$

where b is as defined with reference to formula (1), $X^2$ is halogen and $R^6$ is a protected amino group, in the presence of a strong base and thereafter removing any protecting groups.

Examples of hydroxy protecting groups are $C_{1-6}$-alkyl, for example methyl, and $C_{1-6}$-alkanoyl, for example formyl or acetyl.

These protecting groups can be removed by standard methods, in particular under basic conditions.

Examples of protected amino groups for $R^6$ include phthalimido. In formulae (6), (7) and (9), $X^2$ can be chlorine, bromine or iodine.

Examples of strong bases are alkali metal hydrides, particularly sodium hydride. The reaction is carried out in the presence of a polar solvent for example dimethyl-sulphoxide.

The protected amino group can be converted into amino by standard methods, for example when it is phthalimido by reaction with hydrazine.

The use of protecting groups is discussed in J.F. McOmie, Protective Groups in Organic Chemistry, 1973, Plenum Press, IBSN 0-306-30717-0.

Compounds of formula (3) are known or can be made by known methods as described in for example British Patent Application No. 2067987A.

Compounds of formulae (6) to (9) are known or can be made by known methods.

The histamine $H_1$-antagonist activity of the compounds of formula (1) can be demonstrated in vitro in the guinea pig ileum test. In this test an isolated portion of the guinea pig ileum is secured under tension (500 mg) between an anchorage and a transducer in a 10 ml tissue bath and immersed in magnesium free Tyrode solution with constant aeration at a temperature of 30 °C. The output from the transducer is amplified. The amplified output is in turn fed to a flat bed recorder. Measured amounts of histamine are added to the tissue bath so that the histamine concentration increases step-wise until the force of the contraction reaches a maximum. The tissue bath is washed out and filled with fresh magnesium free Tyrode solution containing compound under test. The solution is left in contact with the tissue for 8 min. and measured amounts of histamine are added again until a maximum contraction is recorded. The assay is repeated with increasing concentrations of test compound and the dose of histamine giving 50 % of maximum contraction is noted. A dose ratio (DR) was calculated by comparing the concentrations of histamine required to produce 50 % maximum response in the absence and in the presence of the antagonist. A plot of Log DR-1 against Log D (the concentration of compound under test) is made and the point of intersection with the Log (DR-1) ordinate is taken as the measure of the activity ($pA_2$ value). The compounds of the Examples have $pA_2$ values greater than 7.

The activity of compounds of formula (1) as histamine $H_1$-antagonists can be demonstrated in vivo by the inhibition of histamine induced bronchoconstriction. Guinea pigs of either sex are anaesthetised by intraperitoneal injection of sodium pentobarbitone, 90 mg/kg. The trachea is cannulated. The animal is respired artificially with a fixed volume or air just adequate to inflate the lungs. The pressure needed to inflate the lungs is monitored from the respiratory system using a low pressure transducer. Intravenous injection of histamine causes dose-dependent increases in the pressure to inflate the lungs reflecting the bronchoconstrictor action of histamine. Responses to histamine can be antagonised using histamine $H_1$-receptor antagonists.

Dose-response curves to histamine are established at 20, 40, 80, 160 and 320 nmols/kg. Antagonists are then administered by intravenous injection and 5 minutes later a new histamine dose-response curve is established increasing the doses of histamine as necessary. The effect of the antagonist can be quantified by the displacement, to the right, of the histamine dose-response curve, expressed as a dose-ratio. A series of doses of antagonists may be given to each animal allowing calculation of dose-ratios for each dose of antagonist.

In order to use the compounds of the invention as histamine $H_1$-antagonists, they can be formulated as pharmaceutical compositions in accordance with standard pharmaceutical procedure.

The invention also includes pharmaceutical compositions comprising a compound of formula (1) or a pharmaceutically acceptable salt thereof and a pharmaceutically acceptable carrier.

Compounds of formula (1) and their pharmaceutically acceptable salts can be administered topically or systemically.

Topical formulations for administration tc the skin include lotions and creams. Topical formulations for administration to the respiratory tract include solutions for application via a nebulizer or as an aerosol, or a microfine insufflatable powder. The active ingredient is an insufflatable powder has a small particle size i. e. less than 50 microns and preferably less than 10 microns. The active material is co-presented with a solid carrier for example lactose which has a particle size of less than 50 microns.

Systemic administration can be achieved by rectal, oral or parenteral administration. A typical suppository formulation comprises the active compound with a binding agent and/or lubricating agent for example gelatine or cocoa butter or other low melting vegetable waxes or fats. Typical parenteral compositions consist of a solution or suspension of the active material in a sterile aqueous carrier or parenterally acceptable oil.

Compounds of formula (1) which are active when given orally can be formulated as syrups, tablets, capsules and lozenges. A syrup formulation generally consists of a suspension or solution of the

compound in a liquid carrier for example ethanol, glycerine or water with a flavouring or colouring agent. Where the composition is in the form of a capsule, the solid in granular form optionally with a binding agent is encased in a gelatin shell. Where the composition is in the form of a tablet, any suitable pharmaceutical carrier routinely used for preparing solid formulations can be used. Examples of such carriers include magnesium stearate, starch, lactose, glucose, sucrose, and cellulose. Preferably the composition is in unit dose form for example a tablet, capsule or metered aerosol so that the patient may administer to himself a single dose.

Where appropriate, small amounts of bronchodilators and anti-asthmatics for example sympathomimetic amines particularly isoprenaline, isoetharine, salbutamol, phenylephrine and ephedrine ; xantihine derivatives particularly theophylline and aminophylline ; and corticosteroids particularly prednisolone and adrenal stimulants particularly ACTH can be included. As in common practice, the compositions will usually be accompanied by written or printed directions for use in the medical treatment concerned, in this case as a histamine $H_1$-antagonist for treatment of, for example, asthma, hayfever, rhinitis or allergic eczema.

Each dosage unit for oral administration contains preferably from 5 to 200 mg of compound of formula (1) or a pharmaceutically acceptable salt thereof calculated as the free base.

The pharmaceutical compositions of the invention will normally be administered to a man for the treatment of rhinitis, hayfever, bronchial asthma or allergic ecxema. An adult patient will receive an oral dose of between 15 mg and 400 mg and preferably between 15 mg and 200 mg or an intravenous, subcutaneous or intramuscular dose of between 1 mg and 50 mg, and preferably between 1 mg and 10 mg of a compound of formula (1) or a pharmaceutically acceptable salt thereof calculated as the free base, the composition being administered 1 to 4 times per day.

The following Examples illustrate the invention.

## Example 1

i) A solution of 3-anilinopropionitrile (15g) in ether (450 ml) was added to a stirred suspension of lithium aluminium hydride (12 g) in ether (450 ml) under nitrogen, over 1 hr., maintaining the temperature at ca. 20 °C. The mixture was stirred for 2 hr. at room temperature and then cooled to 12°-15 °C. A mixture of water (12 ml) and tetrahydrofuran (58 ml) was added dropwise to the reaction mixture, followed by sodium hydroxide solution (4N, 12 ml) and water (35 ml). This mixture was strirred for 45 min. and then allowed to stand overnight at room temperature. The resulting solid was removed by filtration. The filtrate was dried over potassium carbonate and the solvent was evaporated to give 3-anilinoproppylamine (13.7 g ; 89 %), which was used without further purification.

ii) Sodium hydride (1.0 g) was dissolved in dimethylsulphoxide (25 ml) at 70°-75 °C. under nitrogen. The solution was cooled and to it was added 3-anilinopropylamine (2.85 g) in dimethylsulphoxide (10 ml) at room temperature. A solution of 2-chloromethylpyridine hydrochloride (3.42 g) in dimethylsulphoxide (15 ml) was added dropwise maintaining the temperature of the reaction mixture in the range 25°-35 °C. After 1.5 hr. the reaction mixture was diluted with water (70 ml) and the mixture was extracted with chloroform. The chloroform extracts were washed with 2N hydrochloric acid and the aqueous layer adjusted to pH 4 and then 6, each time extracting with chloroform. The pH was raised to 11-13 and then extracted with ether. The ether extracts were evaporated to dryness and the residue chromatographed (silica gel, chloroform/methanolic ammonia 60 : 1) to give 3)[N-phenyl-N-(2-pyridylmethyl)amino]propylamine as an oil (0.97 g ; 21 %).

iii) A solution of 3-[N-phenyl-N-(2-pyridylmethyl)-amino]propylamine (0.92 g) in methanol (15 ml) was added dropwise over 40 minutes to a stirred suspension of 3,4-dimethoxy-1,2,5-thiadiazole-1-oxide (0.62 g) in methanol (30 ml) at 0°-5 °C. After allowing the mixture to stand for a further 3 hr. at 10 °C. the mixture was cooled to 0°-5 °C. and methanol saturated with ammonia (15 ml) was added dropwise to it. This mixture was then allowed to stand at room temperature for 2.5 days. The solvent was evaporated and the residue was chromatographed (silica gel, chloroform/methanol 20 : 1). The fractions which contained the required product were collected and the solvent was evaporated. The residue was crystallised twice from ethanol to give 3-[3(N-phenyl-N-pyrid-2-ylmethylamino)propylamino]-4-amino-1,2,5-thiadiazole-1-oxide (0.47 g. 35 %) m.p. 190°-192 °C.

$C_{17}H_{20}N_6OS$

| | | | | |
|---|---|---|---|---|
| Found : | C 57.19, | H 5.64, | N 23.36, | S 9.18 |
| Requires : | C 57.28, | H 5.66, | N 23.58, | S 9.00 |

## Example 2

i) Substituting 4-chloromethylpyridine hydrochloride (4.91g) for 2-chloromethylpyridine hydrochloride and using corresponding molar proportions of other reagents in the method of Example 1(ii) gave, after medium pressure chromatography (silica gel, chloroform/methanolic ammonia 60 : 1), 3-[N-phenyl-N-(4-pyridylmethyl)amino]propylamine as an oil (0.66 g ; 14 %).

ii) Substituting 3-[N-phenyl-N-(4-pyridylmethyl)-amino]propylamine (0.63 g) for 3-[N-phenyl-N-(2-pyridylmethyl)amino]propylamine and using corresponding molar proportions of other reagents in the method of Example 1(iii) gave, after crystallisation from ethanol, 3-[3-(N-phenyl-N-pyrid-4-yl-methylamino)propylamino]-4-amino-1,2,5-thiadiazole-1-oxide (0.34 g ; 37 %) m.p. 96°-99 °C.

$$C_{17}H_{20}N_6OS$$

Found : C 56.98, H 5.72, N 23.41, S 8.88
Requires : C 57.28, H 5.66, N 23.58, S 9.00

Example 3

i) Substituting 3-chloromethylpyridine hydrochloride (10.49 g) for 2-chloromethylpyridine hydrochloride and using corresponding molar proportions of other reagents in the method of Example 1(ii) gave, after medium pressure chromatogpraphy (silical gel, chloroform/methanolic ammonia 24 : 1), 3-[N-phenyl-N-(3-pyridylmethyl)amino]propylamine as an oil (6.22 g ; 48 %).

ii) Substituting 3-[N-phenyl-N-(3-pyridylmethyl)-amino]propylamine (1.30 g) for 3-(N-phenyl-N-(2-pyridylmethyl)amino]propylamine and using corresponding molar proportions of other reagents in the method of Example 1(iii) gave, after crystallisation from chloroform, 3-[3-(N-phenyl-N-pyrid-3-yl-methylamino)propylamino]-4-amino-1,2,5-thiadiazole-1-oxide (0.60 g ; 31 %) m.p. 125°-128 °C. (decomp.).

$$C_{17}H_{20}N_6OS.1.14H_2O$$

Found : C 53.31, H 5.94, N 21.77, S 8.41
Requires : C 53.30, H 5.87, N 21.94, S 8.37

Example 4

A pharmaceutical composition for oral administration is prepared containing

| | | % by weight |
|---|---|---|
| A | 3-[3-(N-phenyl-N-pyrid-4-ylmethyl-amino)propylamino]-4-amino- | 55 |
| A | 1,2,5-thiadiazole-1-oxide Dibasic calcium phosphate dihydrate | 20 |
| A | Approved coloring agent | 0.5 |
| | Polyvinylpyrrolidone | 4.0 |
| B | Microcrystalline Cellulose | 8.0 |
| B | Maize Starch | 8.0 |
| B | Sodium glycollate | 4.0 |
| B | Magnesium Stearate | 0.5 |

by mixing together the ingredients A (substituting lactose or microcrystalline cellulose for dibasic calcium phosphate dihydrate if desired), adding a concentrated solution of polyvinylpyrrolidone and granulating, drying and screening the dried granules ; adding the ingredients B to the dried granules and compressing the mixture into tablets containing 5 mg, 25 mg or 50 mg of the free base.

Example 5

A pharmaceutical composition for injectable administration is prepared by forming a solution of 3-[3-(N-phenyl-N-pyrid-4-ylmethylamino)propylamino]-4-amino-1,2,5-thiadiazole-1-oxide in sterile water to give a 1 to 5 % w/w solution. The solution is clarified by filtration and filled into vials which are sealed and sterilised. A suitable vial contains 2 ml of the solution.

**Claims :** (for the Contracting States : BE, CH, DE, FR, GB, IT, LI, LU, NL, SE)

1. A compound of formula (1)

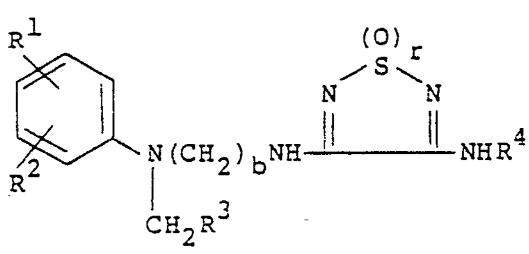

(1)

and pharmaceutically acceptable salts thereof, where

$R^1$ and $R^2$ are the same of different and are hydrogen, $C_{1-6}$-alkyl, $C_{1-6}$-alkoxy or halogen ;

$R^3$ is optionally substituted pyridyl, where the optional substituents are one or more $C_{1-6}$-alkyl, $C_{1-6}$-alkoxy, or hydroxy groups or halogen atoms ;

b is from 2 to 4

$R^4$ is hydrogen or $C_{1-6}$-alkyl, and

r is 1 or 2.

2. A compound as claimed in claim 1 where $R^1$ and $R^2$ are both hydrogen.

3. A compound as claimed in claim 1 or claim 2 where b is 3.

4. A compound as claimed in claim 3 where $R^3$ is 2-pyridyl or 4-pyridyl.

5. A compound as claimed in any one of claims 1 to 4 where $R^4$ is hydrogen.

6. A compound as claimed in any one of claims 1 to 5 where r is 1.

7. A compound as claimed in any one of claims 1 to 6 which is :

3-[3-(N-phenyl-N-pyrid-2-ylmethylamino)propylamino)]-4-amino-1,2,5-thiadiazole-1-oxide,

3-[3-(N-phenyl-N-pyrid-4-ylmehtylamino)propylamino)]-4-amino-1,2,5-thiadiazole-1-oxide, or

3-[3-(N-phenyl-N-pyrid-3-ylmethylamino)propylamino)]-4-amino-1,2,5-thiadiazole-1-oxide.

8. A process for preparing a compound as claimed in any one of claims 1 to 7 which comprises reacting a compound of formula (2)

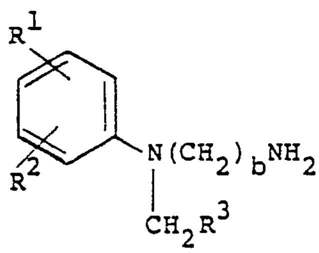

(2)

where $R^1$, $R^2$, $R^3$ and b are as defined with reference to formula (1), with a compound of formula (3)

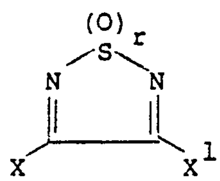

(3)

where X is a group displaceable with an amine and $X^1$ is a group of formula $NHR^4$ or a group displaceable with an amine, where $R^4$ is as defined with reference to formula (1) ; and thereafter, where $X^1$ is a group displaceable with an amine, reacting with an amine of formula (4)

$$R^4NH_2 \qquad (4)$$

where $R^4$ is as defined with reference to formula (1) ; and optionally converting a compound of formula (1) so obtained into a pharmaceutically acceptable acid addition salt.

9. A pharmaceutical composition comprising a compound as claimed in any one of claims 1 to 7 or a pharmaceutically acceptable salt thereof and a pharmaceutically acceptable carrier.

10. A compound as claimed in any one of claims 1 to 7 for use as a histamine $H_1$-antagonist.

**Claims** (for the Contracting State AT)

1. A process for preparing a compound of formula (1)

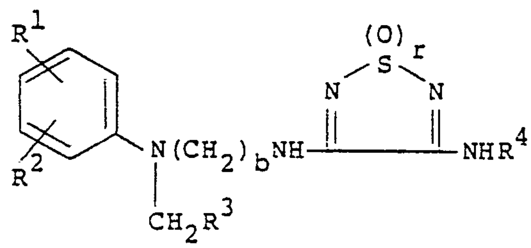

(1)

and pharmaceutically acceptable salts thereof, where

$R^1$ and $R^2$ are the same or diferent and are hydrogen, $C_{1-6}$-alkyl, $C_{1-6}$-alkoxy or halogen ;

$R^3$ is optionally substituted pyridyl, where the optinal substituents are one or more $C_{1-6}$-alkyl, $C_{1-6}$-alkoxy, or hydroxy groups or halogen atoms ;

b is from 2 to 4

$R^4$ is hydrogen or $C_{1-6}$-alkyl, and

r is 1 or 2 ;

which comprises reacting a compound of formula (2)

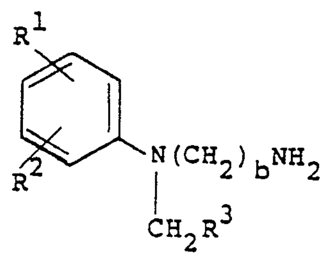

(2)

where $R^1$, $R^2$, $R^3$ and b are as defined with reference to formula (1), with a compound of formula (3)

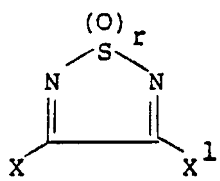

(3)

where X is a group displaceable with an amine and $X^1$ is a group of formula $NHR^4$ or a group displaceable with an amine, where $R^4$ is as defined with reference to formula (1) ; and thereafter, where $X^1$ is a group displaceable with an amine, reacting with an amine of formula (4)

$$R^4NH_2 \quad (4)$$

where $R^4$ is as defined with reference to formula (1) ; and optionally converting a compound of formula (1) so obtained into a pharmaceutically acceptable acid addition salt.

2. A process as claimed in claim 1 where $R^1$ and $R^2$ are both hydrogen.
3. A process as claimed in claim 1 or claim 2 where b is 3.
4. A process as claimed in claim 3 where $R^3$ is 2-pyridyl or 4-pyridyl.
5. A process as claimed in any one of claims 1 to 4 where $R^4$ is hydrogen.
6. A process as claimed in any one of claims 1 to 5 where r is 1.
7. A process as claimed in any one of claims 1 to 6 for preparing :
3-[3-(N-phenyl-N-pyrid-2-ylmethylamino) propylamino]-4-amino-1,2,5-thiadiazole-1-oxide,
3-[3-(N-phenyl-N-pyrid-4-ylmethylamino) propylamino]-4-amino-1,2,5-thiadiazole-1-oxide, or
3-[3-(N-phenyl-N-pyrid-3-ylmethylamino) propylamino]-4-amino-1,2,5-thiadiazole-1-oxide.
8. A process for preparing a pharmaceutical compositon comprising a compound of formula (1) in claim 1 which comprises bringing into association a compound of formula (1) in claim 1 or a pharmaceutically acceptable salt thereof and a pharmaceutically acceptable carrier.
9. A process according to any one of claims 1 to 7 in which X and $X^1$ are QS-, QSO-, $QSO_2$- or QO-, where Q is $C_{1-6}$-alkyl, aryl or aralkyl, halogen or nitroamino.
10. A process according to claim 9 in which X and $X^1$ are both methoxy.

**Patentansprüche** (für die Vertragsstaaten : BE, CH, DE, FR, GB, IT, LI, LU, NL, SE)

1. Eine Verbindung der Formel (1)

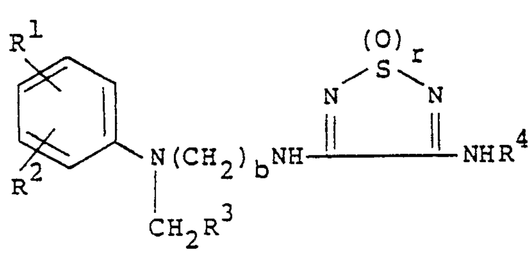

(1)

und pharmazeutisch verträgliche Salze davon, in der

$R^1$ und $R^2$ gleich oder verschieden sind und Wasserstoffatome, $C_{1-6}$-Alkyl- oder $C_{1-6}$-Alkoxyreste oder Halogenatome bedeuten ;

$R^3$ eine gegebenenfalls substituierte Pyridylgruppe darstellt, in der die Substituenten ein oder mehrere $C_{1-6}$-Alkyl-, $C_{1-6}$-Alkoxy- oder Hydroxyreste oder Halogenatome sind ;

b 2 bis 4 ist ;

$R^4$ ein Wasserstoffatom oder einen $C_{1-6}$-Alkylrest bedeutet, und

r 1 oder 2 ist.

2. Verbindung nach Anspruch 1, in der $R^1$ und $R^2$ beide Wasserstoffatome sind.

3. Verbindung nach Anspruch 1 oder Anspruch 2, in der b 3 ist.

4. Verbindung nach Anspruch 3, in der $R^3$ eine 2-Pyridyloder 4-Pyridylgruppe ist.

5. Verbindung nach einem der Ansprüche 1 bis 4, in der $R^4$ ein Wasserstoffatom ist.

6. Verbindung nach einem der Ansprüche 1 bis 5, in der r 1 ist.

7. Verbindung nach einem der Ansprüche 1 bis 6, nämlich :

3-[3-(N-Phenyl-N-pyrid-2-ylmethylamino) propylamino]-4-amino-1,2,5-thiadiazol-1-oxid,

3-[3-(N-Phenyl-N-pyrid-4-ylmethylamino) propylamino]-4-amino-1,2,5-thiadiazol-1-oxid, oder

3-[3-(N-Phenyl-N-pyrid-3-ylmethylamino) propylamino]-4-amino-1,2,5-thiadiazol-1-oxid.

8. Verfahren zur Herstellung einer Verbindung nach einem der Ansprüche 1 bis 7, umfassend die Umsetzung einer Verbindung der Formel (2)

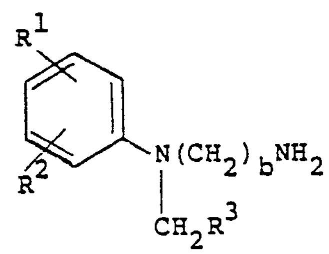

(2)

in der $R^1$, $R^2$, $R^3$ und b wie mit Bezug auf die Formel (1) definiert sind, mit einer Verbindung der Formel (3)

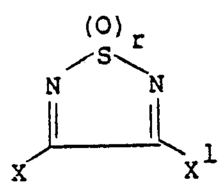

(3)

in der X eine durch eine Aminogruppe verdrängbare Gruppe ist und $X^1$ eine Gruppe der Formel $NHR^4$ oder eine durch eine Aminogruppe verdrängbare Gruppe darstellt, in der $R^4$ wie mit Bezug auf Formel (1) definiert ist, und anschließend, wenn $X^1$ eine durch eine Aminogruppe ver drängbare Gruppe darstellt, Umsetzung mit einem Amin der Formel (4)

$$R^4NH_2 \qquad (4)$$

in der $R^4$ wie mit Bezug auf Formel (1) definiert ist, und gegebenenfalls Umwandlung einer so erhaltenen Verbindung der Formel (1) in ein pharmazeutisch verträgliches Säureadditionssalz.

9. Arzneimittel, umfassend eine Verbindung nach einem der Ansprüche 1 bis 7 und ein pharmazeutisch verträgliches Salz.

10. Verbindung nach einem der Ansprüche 1 bis 7 zur Verwendung als Histamin $H_1$-Antagonist.

**Patentansprüche** (für den Vertragsstaat AT)

1. Verfahren zur Herstellung einer Verbindung der Formel (1)

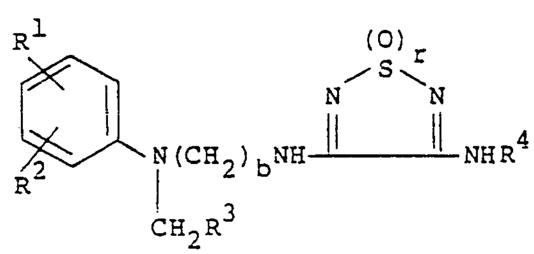

(1)

und pharmazeutisch verträgliche Salze davon, in der

$R^1$ und $R^2$ gleich oder verschieden sind und Wasserstoffatome, $C_{1-6}$-Alkyl- oder $C_{1-6}$-Alkoxyreste oder Halogenatome bedeuten ;

$R^3$ eine gegebenenfalls substituierte Pyridylgruppe darstellt, in der die Substituenten ein oder mehrere $C_{1-6}$-Alkyl-, $C_{1-6}$-Alkoxy- oder Hydroxyreste oder Halogenatome sind ;

b 2 bis 4 ist ;

$R^4$ ein Wasserstoffatom oder einem $C_{1-6}$-Alkylrest bedeutet, und

r 1 oder 2 ist,

umfassend die Umsetzung einer Verbindung der Formel (2)

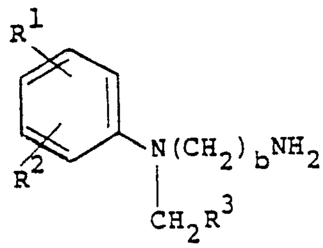

(2)

in der $R^1$, $R^2$, $R^3$ und b wie mit Bezug auf die Formel (1) definiert sind, mit einer Verbindung der Formel (3)

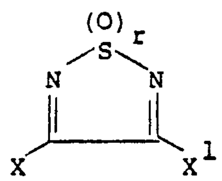

(3)

in der X eine durch eine Aminogruppe verdrängbare Gruppe ist und $X^1$ eine Gruppe der Formel $NHR^4$ oder eine durch eine Aminogruppe verdrängbare Gruppe darstellt, in der $R^4$ wie mit Bezug auf Formel (1) definiert ist, und anschließend, wenn $X^1$ eine durch eine Aminogruppe verdrängbare Gruppe darstellt, Umsetzung mit einem Amin der Formel (4)

$$R^4NH_2 \qquad (4)$$

in der $R^4$ wie mit Bezug auf Formel (1) definiert ist, und gegebenenfalls Umwandlung einer so erhaltenen Verbindung der Formel (1) in ein pharmazeutisch verträgliches Säureadditionssalz.

2. Verfahren nach Anspruch 1, in der $R^1$ und $R^2$ beide Wasserstoffatome sind.

3. Verfahren nach Anspruch 1 oder Anspruch 2, in dem b 3 ist.

4. Verfahren nach Anspruch 3, in dem $R^3$ eine 2-Pyridyl- oder 4-Pyridylgruppe ist.

5. Verfahren nach einem der Ansprüche 1 bis 4, in dem $R^4$ ein Wasserstoffatom ist.

6. Verfahren nach einem der Ansprüche 1 bis 5, in dem r 1 ist.

7. Verfahren nach einem der Ansprüche 1 bis 6 zur Herstellung von :

3-[3-(N-Phenyl-N-pyrid-2-ylmethylamino) propylamino]-4-amino-1,2,5-thiadiazol-1-oxid,

3-[3-(N-Phenyl-N-pyrid-4-ylmethylamino) propylamino]-4-amino-1,2,5-thiadiazol-1-oxid, oder

3-[3-(N-Phenyl-N-pyrid-3-ylmethylamino) propylamino]-4-amino-1,2,5-thiadiazol-1-oxid.

8. Verfahren zur Herstellung eines Arzneimittels, umfassend eine Verbindung der Formel (1) von Anspruch 1, das Zusammenbringen einer Verbindung der Formel (1) von Anspruch 1 oder eines pharmazeutisch verträglichen Salzes davon mit einem pharmazeutisch verträglichen Träger umfaßt.

9. Verfahren nach einem der Ansprüche 1 bis 7, in dem X und $X^1$ Reste der Formel QS-, QSO-, $QSO_2$-, oder QO- darstellen, in den Q einen $C_{1-6}$-Alkyl-, Aryl- oder Aralkylrest, ein Halogenatom oder eine Nitroaminogruppe bedeutet.

10. Verfahren nach Anspruch 9, in dem X und $X^1$ beide Methoxygruppen bedeuten.

**Revendications** (pour les Etats contractants : BE, CH, DE, FR, GB, IT, LI, LU, NL, SE)

1. Composé de formule (1)

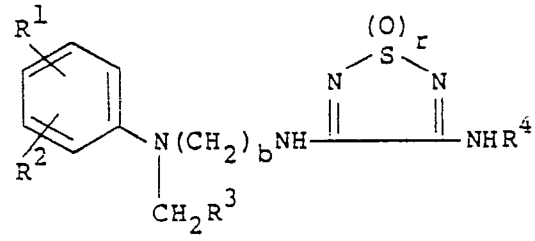

(1)

et ses sels pharmaceutiquement acceptables, où

$R^1$ et $R^2$ sont identiques ou différents et sont de l'hydrogène, un alkyle en $C_{1-6}$, un alkoxy en $C_{1-6}$ ou un halogène ;

$R^3$ est pyridyle éventuellement substitué où les substituants facultatifs sont un ou plusieurs radicaux alkyle en $C_{1-6}$, alkoxy en $C_{1-6}$ ou des groupements hydroxy ou des atomes d'halogène ;

b est compris entre 2 et 4 ;

$R^4$ est de l'hydrogène ou un alkyle en $C_{1-6}$, et

r est 1 ou 2.

2. Composé tel que revendiqué dans la revendication 1 ou $R^1$ et $R^2$ sont tous deux de l'hydrogène.

3. Composé tel que revendiqué dans la revendication 1 ou dans la revendication 2 ou b est 3.

4. Composé tel que revendiqué dans la revendication 3 où $R^3$ est 2-pyridyle ou 4-pyridyle.

5. Composé tel que revendiqué dans l'une quelconque des revendications 1 à 4 ou $R^4$ est de l'hydrogène.

6. Composé tel que revendiqué dans l'une quelconque des revendications 1 à 5 où r est 1.

7. Composé tel que revendiqué dans l'une quelconque des revendications 1 à 6 qui est :

3-[3-(N-phényl-N-pyrid-2-ylméthylamino) promylamino]-4-amino-1,2,5-thiadiazole-1-oxyde,

3-[3-(N-phényl-N-pyrid-4-ylméthylamino) propylamino]-4-amino-1,2,5-thiadiazole -1-oxyde, ou

3-[3-(N-phényl-N-pyrid-3-ylméthylamino) propylamino]-4-amino-1,2,5-thiadiazole-1-oxyde.

8. Procédé pour préparer un composé tel que revendiqué dans l'une quelconque des revendications 1 à 7 qui comprend la réaction d'un composé de formule (2)

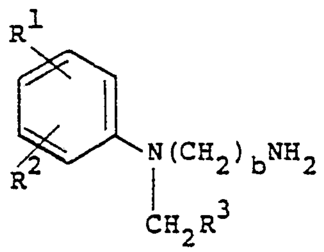

(1)

où $R^1$, $R^2$, $R^3$ et b sont tels que définis par référence à la formule (1), avec un composé de formule (3)

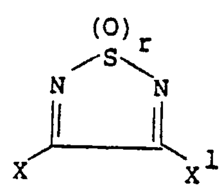

(2)

où X est un groupement déplaçable avec une amine et $X^1$ est un groupement de formule $NHR^4$ ou un groupement déplaçable avec une amine, où $R^4$ est tel que défini par référence à la formule (1) ; et ensuite, lorsque $X^1$ est un groupement déplaçable par une amine, la réaction avec une amine de formule (4)

$$R^4NH_2 \qquad (4)$$

où $R^4$ est tel que défini par référence à la formule (1) et, facultativement, la conversion d'un composé de formule (1) ainsi obtenu en un sel d'addition acide pharmaceutiquement acceptable.

9. Composition pharmaceutique comprenant un composé tel que revendiqué dans l'une quelconque des revendications 1 à 7 ou un sel pharmaceutiquement acceptable de celui-ci et un support pharmaceutiquement acceptable, comme antagoniste $H_2$ de l'histamine.

10. Composé tel que revendiqué dans l'une quelconque des revendications 1 à 7 pour application comme antagoniste $H_2$ de l'histamine.

**Revendications** (pour l'Etat contractant AT)

1. Procédé pour préparer un composé de formule (1)

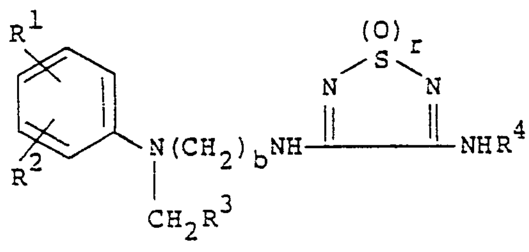

(1)

et ses sels pharmaceutiquement acceptables, où

$R^1$ et $R^2$ sont identiques ou différents et sont de l'hydrogène, un alkyle en $C_{1-6}$-un alkoxy en $C_{1-6}$ ou un halogène ;

$R^3$ est pyridyle facultativement substitué, où les substituants facultatifs sont un ou plusieurs radicaux alkyle en $C_{1-6}$-alkoxy en $C_{1-6}$ ou groupements hydroxy ou atomes d'halogène ;

b est compris entre 2 et 4 ;

$R^4$ est de l'hydrogène ou un alkyle en $C_{1-6}$, et

r est 1 ou 2 ;

qui comprend la réaction d'un composé de formule (2)

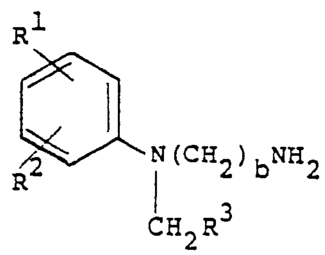

(2)

où $R^1$, $R^2$, $R^3$ et b sont tels que définis par référence à la formule (1) avec un composé de formule (3)

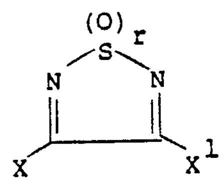

(3)

où X est un groupement déplaçable avec une amine et $X^1$ est un groupement de formule $NHR^4$ ou un groupement déplaçable avec une amine, où $R^4$ est tel que défini par référence à la formule (1) ; et, ensuite, lorsque $X^1$ est un groupement déplaçable avec une amine, la réaction avec une amine de formule (4)

$$R^4NH_2 \quad (4)$$

où $R^4$ est tel que défini par référence à la formule (1) et, facultativement, la conversion d'un composé de formule (1) ainsi obtenu en un sel d'addition acide pharmaceutiquement acceptable.

2. Procédé tel que revendiqué dans la revendication 1 où $R^1$ et $R^2$ sont tous deux de l'hydrogène.

3. Procédé tel que revendiqué dans la revendication 1 ou la revendication 2 où b est 3.

4. Procédé tel que revendiqué dans la revendication 3 où $R^3$ est 2-pyridyle ou 4-pyridyle.

5. Procédé tel que revendiqué dans l'une quelconque des revendications 1 à 4 où $R^4$ est de l'hydrogène.

6. Procédé tel que revendiqué dans l'une quelconque des revendications 1 à 5 où r est 1.

7. Procédé tel que revendiqué dans l'une quelconque des revendications 1 à 6 pour préparer :

3-[3-(N-phényl-N-pyrid-2-ylméthylamino) propylamino]-4-amino-1,2,5-thiadiazole-1-oxyde,

3-[3-(N-phényl-N-pyrid-4-ylméthylamino) propylamino]-4-amino-1,2,5-thiadiazole-1-oxyde, ou

3-[3-(N-phényl-N-pyrid-3-ylméthylamino) propylamino]-4-amino-1,2,5-thiadiazole-1-oxyde.

8. Procédé pour préparer une composition pharmaceutique comprenant un composé de formule (1) dans la revendication 1 qui comprend la mise en association d'un composé de formule (1) dans la revendication 1 ou un sel pharmaceutiquement acceptable de celui-ci et un support pharmaceutiquement acceptable.

9. Procédé suivant l'une quelconque des revendications 1 à 7 dans lequel X et $X^1$ sont QS-, QSO-, $QSO_2$- ou QO-, où Q est alkyle en $C_{1-6}$, aryle ou aralkyle, halogène ou nitroamino.

10. Procédé suivant la revendication 9 dans lequel X et $X^1$ sont tous deux méthoxy.